# EUROPEAN PATENT APPLICATION

(11) **EP 4 600 364 A1**
(43) Date of publication of application: **13.08.2025**
(21) Application number: 23912676.6
(22) Date of filing: 12.12.2023
(51) Int. Cl.: C12N 15/77, C12N 9/10, C12P 13/06, C12R 1/15

(54) **NOVEL POLYNUCLEOTIDE AND METHOD FOR PRODUCING L-ALANINE USING SAME**

(30) Priority: 30.12.2022 KR 20220190977
(71) Applicant: CJ CheilJedang Corporation, Seoul 04560 (KR)
(72) Inventor: KIM, Ju-Yeon, Seoul 04560 (KR); KIM, Min-Jung, Seoul 04560 (KR); LEE, Ji Hye, Seoul 04560 (KR)
(74) Representative: Meissner Bolte Partnerschaft mbB
(86) International application number: PCT/KR2023/020472
(87) International publication number: WO 2024/144001

(57) **Abstract**

The present invention relates to a novel polynucleotide and a method for producing L-alanine using same. Microorganisms incorporating the novel polynucleotide in which the specific position of the promoter region of the gene coding for alaT according to the present invention is altered exhibit a markedly increased ability to produce L-alanine, and thus the novel polynucleotide can be effectively utilized in the efficient production of L-alanine.

## Description

### [TECHNICAL FIELD]

### Cross-reference to related application(s)

The present application claims the benefit of the priority based on Korean patent application No. 10-2022-0190977 filed on December 30, 2022, and the entire contents disclosed in the documents of the corresponding Korean patent application are incorporated as a part of the present description.

The present invention relates to a novel polynucleotide and a method for producing L-alanine using the same, and more specifically, relates to a novel polynucleotide, a recombinant vector comprising the polynucleotide, a host cell transformed with the vector and a method for producing L-alanine using the host cell.

### [BACKGROUND ART]

L-alanine is a colorless or white crystal which is an amino acid has no odor but has a unique sweet taste, and is widely allied in fields such as chemistry, food, medicine and the like. In the food industry, it enhances an umami taste, and has various physiological functions such as promotion of alcohol metabolism, protection of liver functions, and promotion of insulin secretion. It has a function to inhibit browning reaction compared to other amino acids, so it is used as an acidity corrector, and it is utilized in a variety of ways such as medicine of prostatic hypertrophy and sports products and the like. Based on 2020, the annual production of alanine is approximately 500 tons, and the market size is estimated to be 250 million USD or more.

Most food additives or pigment additives are obtained through natural resource extraction, chemical synthesis or biological production, and L-alanine is mainly produced by chemical synthesis or enzymatic conversion. Recently, according to the change in consumers' awareness of focusing on healthy life, natural substances have become more preferred than artificial products despite of higher prices due to low production. Accordingly, various studies beyond the method for producing petroleum-derived L-alanine, for example, efforts to develop a microorganism producing a high concentration of amino acids or a fermentation process technology have been made.

Common methods for producing L-alanine include a method for fermentation using a microorganism such as *Corynebacterium* or *Escherichia coli, Brevibacterium, Lactobacillus* sp. And the like (US 5559016 A).

However, according to the increase in demand of L-alanine, the need for research to produce a high concentration of L-alanine more efficiently comes to the fore.

Accordingly, as a result to develop a microorganism producing a high concentration of L-alanine, the present inventors have developed a novel polynucleotide in which a specific position of a promoter region of a gene encoding alaT is mutated, and have confirmed that the production of L-alanine can be improved using the polynucleotide, thereby completing the present invention.

### [DISCLOSURE]

### [TECHNICAL PROBLEM]

One embodiment of the present invention provides a polynucleotide, in which the nucleotides corresponding to the 88th position and 90th position are substituted with T, respectively, and the nucleotide corresponding to the 91th position is substituted with A, based on the nucleotide sequence represented by SEQ ID NO: 2.

Another embodiment of the present invention provides a vector comprising the polynucleotide; and a vector comprising a gene encoding a target protein operably linked to the polynucleotide.

Other embodiment of the present invention provides a host cell comprising the polynucleotide; and a vector comprising a gene encoding a target protein operably linked to the polynucleotide.

Other embodiment of the present invention provides a method for producing an amino acid comprising culturing the host cell in a medium.

Other embodiment of the present invention provides a composition for producing an amino acid comprising the host cell.

Other embodiment of the present invention provides a use of a polynucleotide, in which the nucleotides corresponding to the 88th position and 90th position are substituted with T, respectively, and the nucleotide corresponding to the 91th position is substituted with A, based on the nucleotide sequence represented by SEQ ID NO: 2, a vector comprising the polynucleotide, or a host cell comprising the vector, for producing an amino acid.

### [TECHNICAL SOLUTION]

This is explained in detail as follows. On the other hand, each description and embodiment disclosed in the present invention can be applied to each other description and embodiment. In other words, all combinations of various elements disclosed in the present invention fall within the scope of the present invention. In addition, the scope of the present application cannot be considered limited by the specific description described below.

The present invention provides a polynucleotide, in which the nucleotides corresponding to the 88th position and 90th position are substituted with T, respectively, and the nucleotide corresponding to the 91th position is substituted with A, based on the nucleotide sequence represented by SEQ ID NO: 2.

In the present description, the term "nucleotide sequence represented by SEQ ID NO: 2" may mean a part of a promoter sequence of a gene encoding aminotransferase.

In the present description, the term "aminotransferase" can be interchangeably used with "aminotransferase", and means an enzyme having a function capable of producing L-alanine from pyruvate reversibly. A cofactor used at that time, L-valine may affect reduction of by-products.

In the present description, the term "L-alanine" is one of essential amino acids, and means an L-amino acid having the chemical formula of HO₂CCH(NH₂)CH₃.

The polynucleotide may have promoter activity.

In the present description, the term "promoter" means a non-translated nucleotide sequence of the upstream of a coding region, comprising a binding site to polymerase and having transcription initiation activity to mRNA of a promoter target gene, that is, a DNA region to which polymerase binds to initiate transcription of the gene. The promoter may be located on the 5' site of the mRNA transcription initiation site. Then, the target gene of the promoter may be a gene encoding aminotransferase, but not limited thereto.

The polynucleotide of the present invention is that a part of the nucleotide sequence represented by SEQ ID NO: 2, that is, the promoter sequence of the gene encoding aminotransferase, is mutated, and specifically, the mutation may be that the nucleotides corresponding to the 88th position and 90th position of the sequence are substituted with T, respectively, and the nucleotide corresponding to the 91th position is substituted with A. Accordingly, the polynucleotide may consist of the nucleotide sequence of SEQ ID NO: 1.

The term "mutation" means a genetically or non-genetically stable phenotypic change, and in the present description, it may be named by being interchangeably used with "mutant".

Specifically, the polynucleotide may have increased promoter activity compared to a polynucleotide comprising no mutation (wild-type polynucleotide). Therefore, it may control (increase) expression of a target gene operably linked to the polynucleotide and activity of a protein encoded by the target gene, and moreover, it may control expression a gene other than the target gene.

The polynucleotide may have activity of increasing production ability (production) of an amino acid, for example, production ability (production) of L-alanine, of a host cell, by being introduced into an appropriate host cell. Therefore, the polynucleotide may be for increasing production ability (production) of an amino acid, and specifically, it may be for increasing production ability (production) of L-alanine.

Specifically, the polynucleotide may consist of the nucleotide sequence of SEQ ID NO: 1.

In addition, the nucleotide sequence of the present invention may be modified by conventionally known mutagenesis, for example, direct evolution and site-directed mutagenesis and the like.

Therefore, the polynucleotide may comprise a polynucleotide comprising a nucleotide sequence having homology of at least one 60% or more, specifically, 70% or more, more specifically, 80% or more, much more specifically, 83% or more, 84% or more, 88% or more, 90% or more, 93% or more, 95% or more, or 97% or more, to the nucleotide sequence of SEQ ID NO: 1. As long as it is a sequence having homology to the sequence, and it is a polynucleotide sequence substantially having same or corresponding biological activity (promoter activity) and/or desired activity (for example, activity of increasing production of L-alanine in a host cell) to the nucleotide sequence of SEQ ID NO: 1, even a case of having a polynucleotide sequence in which some sequences are deleted, modified, substituted or added may also be included in the scope of the present application.

In the present description, the term "identity (or homology)" means a degree of corresponding to a given nucleic acid sequence or amino acid sequence, and may be expressed as a percentage (%). In case of the homology to nucleic acid may be determined using documentary algorithm BLAST (see: Karlin and Altschul, Pro. Natl. Acad. Sci. USA, 90, 5873, 1993) or FASTA by Pearson (see: Methods Enzymol., 183, 63, 1990). Based on this algorithm BLAST, a program called BLASTN or BLASTX has been developed (see: http://www.ncbi.nlm.nih.gov).

In one embodiment, the polynucleotide comprising a specific nucleic acid sequence provided in the present description may be interpreted as comprising a polynucleotide fragment comprising not only the specific nucleic acid sequence or a substantially equivalent nucleic acid sequence thereto, but also a nucleic acid sequence complementary to the specific nucleic acid sequence. Specifically, the complementary polynucleotide may be hybridized at a Tm value appropriately adjustable by those skilled in the art according to the purpose, for example, a Tm value of 55°C, 60°C, 63°C or 65°C, and may be analyzed under the conditions described below: These conditions are described specifically in known documents. For example, a condition in which genes having high complementarity of 60% or more, 70% or more, 80% or more, 85% or more, 90% or more, 91% or more, 92% or more, 93% or more, 94% or more, 95% or more, 96% or more, 97% or more, 98% or more, 99% or more, 99.5% or more, or 99.9% or more are hybridized, and genes having lower complementarity than that are not hybridized, or a condition of washing once, specifically, twice or 3 times, at a salt concentration and a temperature corresponding to 60°C, 1x SSC(saline-sodium citrate buffer), and 0.1%(w/v) SDS (Sodium Dodecyl Sulfate); 60°C, 0.1x SSC, and 0.1% SDS; or 68°C, 0.1x SSC, and 0.1% SDS, which are washing conditions of common southern hybridization, and the like may be listed, but not limited thereto. Hybridization requires that two nucleotides have complementary sequences, but depending on the stringency of hybridization, a mismatch between bases may be allowed. The term "complementary" may be used to describe relationship between nucleotide bases that can be hybridized to each other. For example, in case of DNA, adenosine is complementary to thymine and cytosine is complementary to guanine. The appropriate stringency for hybridizing a polynucleotide depends on the length and degree of complementarity of the polynucleotide, and this is well known in the related art (see Sambrook et al., supra, 9.50-9.51, 11.7-11.8).

In particular, the expression of "consist of the nucleotide sequence of SEQ ID NO: 1" does not exclude cases of addition, and/or deletion, and/or mutation of a nucleotide, which can occur during a process of linking to the target gene with use of a restriction enzyme, when the corresponding polynucleotide is used to be linked to a target gene as a promoter.

For example, the polynucleotide having biological activity (promoter activity) consisting of the nucleotide sequence represented by SEQ ID NO: 1, and/or target activity (for example, activity of increasing production of L-alanine in a host cell), may be comprised without limitation, as long as it is a nucleotide sequence having biological activity (promoter activity) of the present application and/or target activity (for example, activity for increasing production of L-alanine) by being hybridized under stringent conditions with a complementary sequence to all or a part of the nucleotide sequence of SEQ ID NO: 1.

Furthermore, the polynucleotide of the present invention may be operably linked to a gene encoding a target protein.

In the present description, the term "gene expression regulatory sequence" means a sequence which comprises the polynucleotide of the present invention, and can express a target gene operably linked thereto.

In the present description, the term "operably linked" means that the polynucleotide having promoter activity of the present invention is functionally linked to the gene sequence to initiate and mediate transcription of a target gene. Operably linking may be prepared using a gene recombination technology known in the art, and site-specific DNA cutting and ligation may be constructed using cutting and ligation enzymes and the like in the art, but not limited thereto.

In addition, the gene expression regulatory sequence of the present invention may further comprise any operator sequence for regulating transcription, sequence encoding an appropriate mRNA ribosome binding site, and DNA regulating termination of transcription and translation, and the like, in addition to a promoter for conducting transcription of a gene.

For example, a regulatory sequence suitable for prokaryotes may further comprise a ribosome binding site, in addition to the promoter, but not limited thereto. The polynucleotide having promoter activity of the present invention may compose the sequence for regulating gene expression as described above if needed by those skilled in the art.

In the present invention, the target gene refers to a gene encoding a target protein to regulate expression in a microorganism.

For example, it may be a gene involved in amino acid production, but not limited thereto. Specifically, the gene may be a gene encoding an enzyme related to amino acid biosynthesis, but not limited thereto. More specifically, the gene may be a gene encoding aminotransferase, but not limited thereto.

In addition, the present invention provides a vector comprising; a polynucleotide, in which the nucleotides corresponding to the 88th position and 90th position are substituted with T, respectively, and the nucleotide corresponding to the 91th position is substituted with A, based on the nucleotide sequence represented by SEQ ID NO: 2.

The polynucleotide is as described above.

The vector may further comprise a gene encoding a target protein operably linked to the polynucleotide.

In the present description, the term "vector" refers to a DNA product containing a base sequence of a polynucleotide encoding the target protein operably linked to an appropriate regulatory sequence so as to express a target protein in an appropriate host. The regulatory sequence may comprise a promoter capable of initiating transcription, any operator sequence for regulating transcription, a sequence encoding an appropriate mRNA ribosome binding site, and/or a sequence regulating termination of transcription and/or translation. The vector can be transformed into a suitable host cell, and then expressed independently of the genome of the host cell, or integrated into the genome of the host cell.

In the present invention, the vector is not particularly limited as long as it is replicable in a host, and it may be selected from all commonly used vectors. Examples of the commonly used vectors may include a plasmid, cosmid, virus, bacteriophage and the like in a natural state or a recombinant state. For example, as the vector, as the phage vector or cosmid vector, pWE15, M13, MBL3, MBL4, IXII, ASHII, APII, t10, t11, Charon4A, and Charon21A and the like may be used, and as the plasmid vector, pDZ vector, pBR-based, pUC-based, pBluescriptII-based, pGEM-based, pTZ-based, pCL-based, and pET-based and the like may be used. Specifically, pDZ, pACYC177, pACYC184, pCL, pECCG117, pUC19, pBR322, pMW118, pCC1BAC vectors and the like may be exemplified, but not limited thereto.

The available vector in the present description may be a known expression vector and/or a vector for insertion into host cell chromosome of a polynucleotide. The insertion into host cell chromosome of the polynucleotide may be conducted by any method known in the art, for example, homologous recombination or CRISPR system, but not limited thereto. The vector may further comprise a selection marker for confirming whether it is inserted in the chromosome. The selection marker is to select a cell transformed with the vector, that is, to confirm whether the polynucleotide is inserted, and it may be selected from genes giving a selective phenotype such as drug resistance, auxotrophy, resistance to a cytotoxic agent, or expression of a surface protein, and used. Under an environment treated with a selective agent, only cells expressing a selection marker survive or show other phenotypes, so the transformed cells can be selected.

In addition, the present invention provides a host cell comprising the polynucleotide and a gene encoding a target protein operably linked to the polynucleotide.

The polynucleotide and gene encoding a target protein operably linked to the polynucleotide are as described above.

The host cell may be a microorganism, but not limited thereto.

In the present description, the term "microorganism" is a concept which includes all wild-type microorganisms or microorganisms in which genetic modification occurs naturally or artificially, and includes all microorganisms in which a specific mechanism is weakened or enhanced due to causes such that a foreign gene is inserted or activity of an intrinsic gene is enhanced or weakened and the like.

In the present invention, the microorganism may comprise the polynucleotide, and specifically, may comprise the polynucleotide and/or a gene encoding a target protein operably linked to the polynucleotide. Otherwise, the microorganism may comprise a vector comprising the polynucleotide or a gene encoding a gene expression regulatory sequence and a target protein, but not limited thereto. In addition, the polynucleotide, gene encoding the target protein, and vector may be introduced into the microorganism by transformation, but not limited thereto. Moreover, as long as the microorganism can express the gene, it is irrelevant to that the polynucleotide and gene encoding the target protein are positioned on chromosome or positioned outside the chromosome.

The microorganism comprising the polynucleotide and gene encoding the target protein may have increased amino acid production ability, and specifically, it may have increased production ability of L-alanine.

For example, the microorganism may have enhanced aminotransferase.

In the present invention, the microorganism may include, without limitation, as long as it is a microorganism which can operate as a promoter as the polynucleotide having promoter activity of the present invention is introduced.

As one example, the microorganism is a cell or microorganism expressing the polynucleotide of the present application, transformed with a vector comprising the polynucleotide of the present application, and for the purpose of the present application, the microorganism of the present application may include all microorganisms which comprise the mutant of the present application and can produce alanine. For example, the microorganism of the present application may be a recombinant strain with increased L-alanine production ability, as the polynucleotide of the present application is introduced into a natural wild-type microorganism or a microorganism producing L-alanine, and thereby, aminotransferase is enhanced. The recombinant strain with increased L-alanine production ability, may be a microorganism with increased L-alanine production ability, compared to a natural wild-type microorganism or a non-modified microorganism in a promoter region of a gene encoding alaT (namely, a microorganism expressing a promoter of a wild-type alaT encoding gene), but not limited thereto. As its example, the non-modified microorganism in a promoter region of a gene encoding alaT, which is a target strain for comparing an increase in L-alanine production ability, may be a wild-type *Corynebacterium glutamicum* strain, and in one specific embodiment, it may be *Corynebacterium glutamicum* ATCC13869 strain, but not limited thereto.

In one embodiment, the microorganism with increased L-alanine production ability may have increased L-alanine production (or production ability) to about 1% or more, about 2.5% or more, about 5% or more, about 10% or more, about 15% or more, about 20% or more, about 25% or more, about 30% or more, about 35% or more, about 36% or more, about 37% or more, about 38% or more, about 39% or more, about 40% or more, about 41% or more, or about 42% or more (the upper limit is not particularly limited, and for example, it may be about 200% or less, about 150% or less, about 100% or less, about 50% or less), compared to a parent strain before mutated or non-modified microorganism.

In another embodiment, the microorganism with increased L-alanine production ability may have increased L-alanine production (or production ability) to about 1.1 times or more, about 1.12 times or more, about 1.13 times or more, 1.15 times or more, 1.16 times or more, 1.17 times or more, 1.18 times or more, 1.19 times or more, about 1.2 times or more, 1.25 times or more, about 1.3 times or more, about 1.35 times or more, about 1.40 times or more, about 1.41 times or more, or about 1.42 times (the upper limit is not particularly limited, and for example, it may be about 10 times or less, about 5 times or less, about 3 times or less, or about 2 times or less), compared to a parent strain before mutated or non-modified microorganism, but not limited thereto. The term "about" is a range including all of ±0.5, ±0.4, ±0.3, ±0.2, ±0.1 and the like, and includes all numerical values in an equivalent or similar range to a numerical value appearing after the term about, but not limited thereto.

In the present application, "non-modified microorganism" does not exclude strains comprising mutation that can be naturally occurring in a microorganism, and may mean a wild-type strain or natural strain itself, or a strain before traits are changed due to genetic mutation by natural or artificial factors. For example, the non-modified microorganism may mean a strain, in which a polynucleotide in which a promoter region of a gene encoding alaT is mutated disclosed in the present description is not introduced, or before it is introduced. The "non-modified microorganism" may be interchangeably used with "strain before modified", "microorganism before modified", "non-mutated strain", "non-modified strain", "non-mutated microorganism" or "reference microorganism".

Specifically, the microorganism may be a *Corynebacterium* sp. microorganism, and more specifically, *Corynebacterium stationis, Corynebacterium thermoaminogenes, Corynebacterium glutamicum, Brevibacterium flavum, Brevibacterium lactofermentum* and strains prepared therefrom may be comprised, but not limited thereto. Specifically, it may be *Corynebacterium stationis* or *Corynebacterium glutamicum* strain.

In addition, the present invention provides a method for producing an amino acid comprising culturing a host cell comprising a polynucleotide, in which the nucleotides corresponding to the 88th position and 90th position are substituted with T, respectively, and the nucleotide corresponding to the 91th position is substituted with A, based on the nucleotide sequence represented by SEQ ID NO: 2, in a medium.

The polynucleotide and host cell are as described above.

The method, may further comprise recovering the amino acid from the cultured microorganism, cultured product, or both of them, after the culturing.

The amino acid may be L-alanine, but not limited thereto.

The term in the present description, "culture" means growing a microorganism under an appropriately artificially adjusted environmental condition. The method for culturing a microorganism of the present invention may be performed using a culture method of *Corynebacterium glutamicum* widely known in the art. Specifically, examples of the culture method include batch culture, continuous culture and fed-batch culture, but not limited thereto. These various methods are disclosed, for example, in "Biochemical Engineering" (James M. Lee, Prentice-Hall International Editions, pp138-176, 1991), and the like.

The term in the present description, "cultured product" means a substance comprising a medium in which a microorganism is growing or have grown under an appropriately artificially adjusted environmental condition. In a narrow sense, the grown microorganism is not comprised in the cultured product, but it may be comprised in a broad sense. The "cultured product" comprises various substances excreted by the microorganism during growing together with medium components made for culturing a microorganism, and specifically, a target substance, L-alanine is comprised.

The medium used for culture should satisfy requirements of a specific strain in an appropriate way. The culture medium for a *Corynebacterium* sp. strain is known. For example, the microorganism of the present application may be cultured in a common medium containing suitable carbon sources, nitrogen sources, vitamins and the like under an aerobic condition while adjusting temperature, pH and the like. At this time, the carbon sources include carbohydrates such as glucose, fructose, and sucrose, amino acids such as glutamic acid, cysteine and the like. Specifically, natural organic nutrients such as starch hydrolysate, and molasses may be used, and preferably, they are carbohydrates such as glucose, fructose, sterilized pre-treated molasses (namely, molasses converted to reducing sugar) and the like, and other carbon sources in an appropriate amount may be diversely used without limitation, but not limited thereto. As the nitrogen sources, inorganic nitrogen sources such as ammonia; and organic nitrogen sources such as amino acids such as glutamic acid and cysteine, and peptone, meat extract, yeast extract and the like may be used. These nitrogen sources may be used alone or in combination, but not limited thereto. In the medium, as the phosphorus sources, phosphoric acid, potassium dihydrogen phosphate or dipotassium hydrogen phosphate or corresponding sodium-containing salts may be used, but not limited thereto. As the inorganic compounds, magnesium sulfate, iron sulfate, manganese sulfate and calcium chloride and the like may be used, and in addition thereto, amino acids, vitamins, and appropriate precursors and the like may be comprised. These media or precursors may be added to the cultured product in a batch or continuous method, but not limited thereto.

During culture, by adding a compound such as potassium hydroxide, ammonia and phosphoric acid to the cultured product in an appropriate method, the pH of the cultured product may be adjusted. In addition, during culture, foam generation may be inhibited using an antifoaming agent such as fatty acid polyglycol ester. Furthermore, in order to maintain the aerobic condition of the cultured product, oxygen or oxygen-containing gas may be injected to the cultured product. The temperature of the cultured product may be 27°C to 37°C, specifically, 30°C to 33°C. The culture period may be continued until the produced amount of the desired useful substance is obtained, and specifically, it is 20 to 120 hours.

The recovering the amino acid may be collecting a target amino acid from a medium, culture solution, or microorganism using an appropriate method known in the art according to the culture method. For example, the recovering may be performed by at least one method selected from centrifugation, filtration, anion exchange chromatography, crystallization, HPLC and the like, but not limited thereto. The method for producing an amino acid, may additionally comprise a purifying step, before, at the same time as, or after the recovering.

### [ADVANTAGEOUS EFFECTS]

The present invention relates to a novel polynucleotide and a method for producing L-alanine using the same, and a microorganism to which the novel polynucleotide in which a specific position of a promoter region of a gene encoding alaT of the present invention is introduced has significantly increased production ability of L-alanine, and thus, the novel polynucleotide can be usefully used for producing L-alanine efficiently.

### [MODE FOR INVENTION]

Hereinafter, the present invention will be described in more detail by the following examples. However, these examples are intended to illustrate the present invention only, but the scope of the present invention is not limited by these examples.

### Example 1. Construction of strain in which mutation is introduced in a promoter region of alaT gene

A mutant strain, in which a mutation that the 88th nucleotide C is substituted with T, and the 90th nucleotide A is substituted with T, and the 91th nucleotide C is substituted with A, in a promoter sequence of alaT gene is introduced, was to be constructed.

Specifically, in order to introduce the mutation into a wild-type *Corynebacterium glutamicum* (ATCC13869) (substituting the 88th nucleotide C to T, the 90th nucleotide A to T, and the 91th nucleotide C to A, in the promoter region represented by SEQ ID NO: 2), gene fragments comprising the corresponding mutation were obtained through PCR using each primer pair of SEQ ID NO: 3 and SEQ ID NO: 4, and SEQ ID NO: 5 and SEQ ID NO: 6. The PCR conditions were performed by denaturation at 95°C for 5 minutes, and then repeating denaturation at 95°C for 30 seconds, annealing at 55°C for 30 seconds, and polymerization at 72°C for 30 seconds 30 times, and then polymerization at 72°C for 5 minutes. More specifically, a polynucleotide of 500 bp amplified using the primers of SEQ ID NO: 3 and SEQ ID NO: 4, and a polynucleotide of 500 bp amplified using the primers of SEQ ID NO: 5 and SEQ ID NO: 6 were obtained. The obtained two gene fragments were linked to pDCM2 cut with restriction enzymes BamHI and SalI (Korean patent publication No. 10-2020-0136813) using an infusion enzyme to construct one gene substitution vector comprising the alaT promoter mutation, and this was named pDCM2-Pm_alaT. The information of the primer sequences used for constructing the vector was shown in Table 1 below.

**[Table 1]**

| SEQ ID NO: | Primer name | Sequence (5'->3') |
|---|---|---|
| 3 | alaT_AF | CTCGGTACCCGGGGATCCACAGCCTGGT CGCAGTCC |
| 4 | alaT_AR | GTCTGTAGTCATCCGCTCAATTTTGCCA CTTTGTGT |
| 5 | alaT_BF | ACACAAAGTGGCAAAATTGAGCGGATG ACTACAGACA |
| 6 | alaT_BR | GCATGCCTGCAGGTCGACGTTGGCGATC ATGTCACG |

The vector was transformed to *Corynebacterium glutamicum* ATCC13869 by the electric pulse method (Appl. Microbiol. Biotechnol., 1999), and then it was smeared on a complex plate medium containing kanamycin 25 mg/L to secure colonies. After that, through the conventional secondary crossing process, a final strain in which the corresponding mutation was comprised and the introduced vector was removed was obtained. The strain in which the corresponding mutation was introduced was finally confirmed through gene sequence analysis using the primer pair of SEQ ID NO: 7 (5'-ACGTCAAAGCCTGTGCATC-3') and SEQ ID NO: 8 (5'- CAGCACGTCCTTCATCTTCTC-3'). The strain in which the target mutation was introduced was named CJ0021.

### Example 2. Confirmation of L-alanine production of strain in which novel promoter mutant gene is introduced

In order to compare the production ability of L-alanine of the mutant strain CJ0021 strain constructed by Example 1 and the wild-type *Corynebacterium glutamicum* ATCC 13869 strain, they were cultured by the following method.

Specifically, after inoculating the parent strain, *Corynebacterium glutamicum* ATCC 13869 strain, and the mutant strain CJ0021 strain into a 250 mm corner-baffled flask containing a 25 ml production medium, respectively, they were cultured at 200 rpm at 30°C for 46 hours.

After completing the culturing, the concentration of L-alanine in each medium was measured using liquid high-performance chromatography, and the L-alanine concentration in the culture solution for each tested strain was shown in Table 2 below.

**[Table 2]**

| Comparison of L-alanine production ability of *Corynebacterium glutamicum* ATCC13869 strain and *Corynebacterium glutamicum* CJ0021 strain | | |
|---|---|---|
| | ATCC13869 (parent strain) | CJ0021 (mutant strain) |
| L-alanine concentration (g/L) | 5.9 | 8.4 |

As a result, as shown in Table 2, it was confirmed that the *Corynebacterium glutamicum* CJ0021 strain, in which the mutation was introduced, produced L-alanine at a concentration of 8.4 g/L and had about 142% L-alanine production ability compared to the parent strain.

The above result shows that the mutation that the 88th nucleotide C is substituted to T, and the 90th nucleotide A is substituted to T, and the 91th nucleotide C is substituted with A, in the sequence of the promoter region represented by SEQ ID NO: 2 significantly increases the L-alanine production ability of the microorganism, and the promoter in which the 88th nucleotide C is substituted to T, and the 90th nucleotide A is substituted to T, and the 91th nucleotide C is substituted with A, in the sequence of the promoter region represented by SEQ ID NO: 2 can be usefully used for the method for producing L-alanine.

The composition of the media used in Example 2 was as follows.

### <Activation medium>

Beef extract 5 g/L, Polypeptone 10 g/L, Yeast extract 5 g/L, Urea 2 g/L, sodium chloride (NaCl) 2.5 g/L, Agar 20 g/L, Glucose 10 g/L, 10N sodium hydroxide (NaOH) 100 *µ*ℓ /L

### <Seed medium>

Glucose (anhydrous glucose) 20 g/L, Polypeptone 10 g/L, Yeast extract 10 g/L, ammonium sulfate [(NH₄)₂SO₄] 10 g/L, Urea 1.5 g/L, potassium phosphate monobasic (KH₂PO₄) 5.2 g/L, potassium phosphate dibasic (K₂HPO₄) 10.7 g/L, d-Biotin 1.8 mg/L, Thiamine-HCl 9 mg/L, CAPA 9 mg/L, NCA 60 mg/L, magnesium sulfate (MgSO₄) 0.5 g/L

### <Production medium>

Calcium carbonate (CaCO₃) 30 g/L, Sucrose 57 g/L, BM 6 g/L, magnesium sulfate (MgSO₄) 0.5 g/L, (NH₄)₂SO₄ 50 g/L, KH₂PO₄ 1 g/L, Yeast extract 2g/L, Ammonium acetate 6.28 g/L, d-Biotin 0.05 mg/L, Thiamine-HCl 0.1 mg/L, MnSO₄ 6.7 mg/L, FeSO₄ 10 mg/L

## Claims

1. A polynucleotide, in which the nucleotides corresponding to the 88th position and 90th position are substituted with T, respectively, and the nucleotide corresponding to the 91th position is substituted with A, based on the nucleotide sequence represented by SEQ ID NO: 2.

2. The polynucleotide according to claim 1, wherein the polynucleotide consists of the nucleotide sequence of SEQ ID NO: 1.

3. The polynucleotide according to claim 1 or claim 2, wherein the polynucleotide has promoter activity.

4. A recombinant vector comprising the polynucleotide of claim 3; and a gene encoding a target protein operably linked to the polynucleotide.

5. The recombinant vector according to claim 4, wherein the target protein is aminotransferase.

6. *A Corynebacterium* sp. microorganism, comprising the polynucleotide of claim 3; and a gene encoding a target protein operably linked to the polynucleotide.

7. The *Corynebacterium* sp. microorganism according to claim 6, wherein the polynucleotide consists of the nucleotide sequence of SEQ ID NO: 1.

8. The *Corynebacterium* sp. microorganism according to claim 6, wherein the target protein is aminotransferase.

9. The *Corynebacterium* sp. microorganism according to any one claim of claim 6 to claim 8, wherein the *Corynebacterium* sp. microorganism is *Corynebacterium glutamicum.*

10. A method for producing an amino acid, comprising culturing the *Corynebacterium* sp. microorganism according to any one claim of claim 6 to claim 8 in a medium.

11. The method for producing an amino acid according to claim 10, wherein the amino acid is L-alanine.

12. A composition for producing an amino acid comprising the *Corynebacterium* sp. microorganism according to any one claim of claim 6 to claim 8.

13. The composition for producing an amino acid according to claim 12, wherein the amino acid is L-alanine.
